# EUROPEAN PATENT SPECIFICATION

(11) **EP 3 936 165 B1**
(45) Date of publication and mention of the grant of the patent: **05.11.2025**
(21) Application number: 20195013.6
(22) Date of filing: 08.09.2020
(51) Int. Cl.: A61L 27/26, A61L 27/36, A61L 27/48, A61L 27/56

(54) **METHOD FOR PREPARING BONE GRAFT COMPOSITION AND BONE GRAFT COMPOSITION**
VERFAHREN ZUR HERSTELLUNG EINER KNOCHENTRANSPLANTATZUSAMMENSETZUNG UND KNOCHENTRANSPLANTATZUSAMMENSETZUNG
PROCÉDÉ DE PRÉPARATION DE GREFFE OSSEUSE ET COMPOSITION DE GREFFE OSSEUSE

(30) Priority: 10.07.2020 KR 20200085043; 13.07.2020 KR 20200086329
(43) Date of publication of application: 12.01.2022
(73) Proprietor: MedPark Co., Ltd., Busan 46504 (KR)
(72) Inventor: PARK, Jung Bok, 46509 Busan (KR)
(74) Representative: BCKIP Part mbB

(56) References cited:
- EP-A2- 1 642 602
- WO-A1-2014/157985
- WO-A1-93/00050
- KR-A- 20170 015 802
- US-A1- 2012 205 274

## Description

### CROSS-REFERENCE TO RELATED APPLICATION

This application claims priority to Korean Patent Application No. 10-2020-0085043, filed on July 10, 2020, and Korean Patent Application No. 10-2020-0086329, filed on July 13, 2020.

### BACKGROUND OF THE DISCLOSURE

### Technical Field

The present disclosure relates to a method for preparing a bone graft composition, not covered by the claims, and a bone graft composition.

### Description of the Related Art

Various materials and various methods may be used for reconstruction of defective bone. For example, bone graft materials such as bone powders, bone chips, and bone blocks may be used, or methods such as autografting, allografting, and xerografting may be used for reconstruction of defective bone.

Bone graft materials that are used for reconstruction of defective bone may be used in orthopedic surgery, neurosurgery, plastic surgery, otolaryngology, oral and maxillofacial surgery, veterinary medicine (animal hospital), dermatology, dentistry, etc. For example, these materials may be used for bone defects during disc surgery to induce bone regeneration, or may also be used for implant surgery and reconstruction of oral and maxillofacial bone defects.

In addition, these materials may be used for reconstruction of defective bones in humans or animals. In the present disclosure, the use of bone graft materials mainly in dentistry is described, but is not limited thereto.

Meanwhile, Korean Patent No. 10-0401941 discloses technology related to a bone graft material and a preparation method therefor. When a reticular bone is used, which is composed of bioceramic powder and has a three-dimensionally communicating pore structure as disclosed therein, there may be limitations in the effect of bone graft in terms of biocompatibility, mechanical properties, toxicity, and the like. WO2014/157985 A1 discloses a bone graft composition which comprises 30-55 wt% of calcium phosphate compound particles; and 45-70 wt% of a biodegradable hydrogel containing, based on 1000 parts by weight of the hydrogel, 25-35 parts by weight of poloxamer and 0.5-2 parts by weight of hydroxypropyl methylcellulose (HPMC).

### SUMMARY

The present invention provides a bone graft composition as defined in claim 1. An object of the present disclosure is to provide a bone graft composition which has excellent shape retainability and solubility properties by containing hydroxypropyl methylcellulose, wherein the content of the hydroxypropyl methylcellulose is not less than 0.1 parts by weight or less and less than 0.3 parts by weight based on 1 part by weight of the bone graft material.

Another object of the present disclosure is to provide a bone graft composition which has excellent procedural performance by containing a thickener, which has an adhesive property, wherein the thickener is carboxymethyl cellulose, and hydroxypropyl methylcellulose as a crosslinking agent.

Still another object of the present disclosure is to provide a bone graft composition which has excellent bone regeneration ability by containing a thickener, which has an adhesive property, wherein the thickener is carboxymethyl cellulose, and hydroxypropyl methylcellulose as a crosslinking agent.

Also disclosed, but not falling within the scope of the claimed subject-matter, is a method for preparing a bone graft composition, the method including steps of: preparing a bone morphogenetic protein solution by adding a bone morphogenetic protein to a solvent and dissolving the bone morphogenetic protein in the solvent; adsorbing the bone morphogenetic protein onto bone graft material powder; mixing and stirring the bone graft material powder having the bone morphogenetic protein adsorbed thereon, a thickener having an adhesive property, and hydroxypropyl methylcellulose powder as a crosslinking agent capable of bonding with each of the bone graft material powder and the thickener; and freeze-drying the mixture of the bone graft material powder, the thickener and the hydroxypropyl methylcellulose powder, obtained by the mixing and stirring, at low temperature under vacuum, wherein the hydroxypropyl methylcellulose is obtained by heat treatment at a temperature of 500 to 1300°C.

Here, the heat-treatment temperature may be 600 to 800°C.

The present disclosure provides a bone graft composition containing: a bone graft material; a thickener having an adhesive property, wherein the thickener is carboxymethylcellulose, and hydroxypropyl methylcellulose as a crosslinking agent capable of bonding with each of the bone graft material and the thickener.

The content of the hydroxypropyl methylcellulose is not less than 0.1 parts by weight and less than 0.3 parts by weight based on 1 part by weight of the bone graft material.

The content of the thickener is 0.1 parts by weight to 1 part by weight based on 1 part by weight of the bone graft material.

When the bone graft composition is hydrated for application to a bone defect, the volume thereof may be reduced.

When the bone graft composition is hydrated for application to a bone defect, the volume thereof may be reduced by more than 0% and less than 40% at 48 hours after the hydration.

When the bone graft composition is hydrated for application to a bone defect, the volume thereof may be reduced by 10% at 48 hours after the hydration.

### BRIEF DESCRIPTION OF THE DRAWINGS

FIG. 1 is a flow diagram schematically showing a method for preparing a bone graft composition according to one embodiment of the present disclosure.
FIG. 2 shows the volume reduction rate (%) depending on the amount (parts by weight) of hydroxypropyl methylcellulose, calculated based on the result data (Table 1) obtained in an Experimental Example of the present disclosure.

### DESCRIPTION OF SPECIFIC EMBODIMENTS

The scope of the invention is defined by the claims. Embodiments in the description relating to methods for preparing a bone graft composition are not covered by the claims.Any "embodiment" or "example" which is disclosed in the description but is not covered by the claims should be considered as presented for illustrative purposes only. Embodiments of the present disclosure, not falling within the scope of the claimed subject-matter, relate to a method for preparing a bone graft composition, the method being effective for the removal of organic materials from hydroxypropyl methylcellulose, and shortening the process time, and at the same time, reducing immune response.

Embodiments of the present disclosure relate to a bone graft composition which may have excellent effects in terms of procedural performance, bone regeneration, activation of bone formation, biocompatibility, and ease of use by containing a bone graft material, a thickener having an adhesive property, wherein the thickener is carboxymethylcellulose, and hydroxypropyl methylcellulose.

In the following description, the detailed description of publicly-known technology related to the present disclosure will be omitted when it may unnecessarily obscure the subject matter of the present disclosure. In addition, the terms used in the following description are terms defined in consideration of their functions in the present disclosure and may be changed according to the intention of a user or an operator, or according to practice. Therefore, the definitions of these terms should be determined based on the contents throughout the specification.

In the present disclosure, when the repeating unit, compound or resin represented by a formula includes isomers thereof, the corresponding formula representing the repeating unit, compound or resin means a representative formula that also represents the isomers.

**A** bone graft composition of the present disclosure contains a bone graft material, a thickener, wherein the thickener is carboxymethylcellulose, and hydroxypropyl methylcellulose. The bone graft composition may be implanted into a bone defect, and may be used to restore the bone defect by filling the bone defect with the composition or applying the composition to the bone defect.

As used herein, "filling" or "applying" includes not only a case in which the composition is applied to the bone defect in a state in which it has no rigidity, but also a case in which the composition is applied to the bone defect in a state in which it has rigidity. "Imparting rigidity to the bone graft composition and then applying the composition to the bone defect" may mean that the composition is provided in a rigid form having a shape corresponding to the bone defect by a shape forming device (e.g., a 3D printer, etc.) and then applied to the bone defect.

The bone graft material may be natural bone, for example, autogenous bone, allogeneic bone, or xenogenic bone. When the natural bone is used, it may exhibit an excellent bone formation effect, because it has excellent biocompatibility and also has good wettability and hygroscopicity due to a large number of pores contained therein. In addition, the bone graft material may also be used for reconstruction of defective bone in orthopedic surgery, neurosurgery, plastic surgery, otolaryngology, oral and maxillofacial surgery, veterinary medicine (animal hospital), dermatology, dentistry, etc.

In addition, the bone graft material may be used for reconstruction of defective bones in humans or animals. The use of the bone graft material mainly in dentistry is described below, but is not limited thereto.

The method for preparing a bone graft composition according to an embodiment of the present disclosure, not falling within the scope of the claimed subject-matter, includes steps of: preparing a bone morphogenetic protein solution by adding a bone morphogenetic protein to a solvent and dissolving the bone morphogenetic protein in the solvent; adsorbing the morphogenetic protein onto bone graft material powder; mixing and stirring the bone graft material powder having the bone morphogenetic protein adsorbed thereon, a thickener having an adhesive property, and hydroxypropyl methylcellulose powder as a crosslinking agent capable of bonding with each of the bone graft material powder and the thickener; and freeze-drying the mixture of the bone graft material powder, the thickener and the hydroxypropyl methylcellulose powder, obtained by the mixing and stirring, at low temperature under vacuum, wherein the hydroxypropyl methylcellulose is obtained by heat treatment at a temperature of 500 to 1300°C.

More preferably, the hydroxypropyl methylcellulose may be obtained by heat treatment at a temperature of 600 to 800°C.

If the heat-treatment temperature is lower than 500°C, it is difficult to remove organic materials. In addition, since the heat-treatment temperature is low, the time required for the process is long, resulting in very low process efficiency. Moreover, immune response may be induced by low-temperature heat treatment.

If the heat-treatment temperature is higher than 1,300°C, the particles become highly dense and have a very small volume, and thus the efficiency versus weight thereof significantly decreases. Furthermore, the particles become sharp, and thus operability and shape retainability are very poor.

Therefore, when the hydroxypropyl methylcellulose is heat-treated at a heat-treatment temperature of 600°C to 1,300°C, more preferably 600°C to 800°C, it is possible to obtain a bone graft composition having excellent volume ratio, operability, hydration feeling, and solubility. The bone graft composition according to an embodiment of the present disclosure may have adhesion to a bone defect and bone regeneration ability by containing a thickener and hydroxypropyl methylcellulose.

When the bone graft material is used alone, it easily disperses in water or blood. For this reason, a viscous thickener may be used to ensure the formability of the bone graft material. However, if the thickener alone is mixed with the bone graft material, the thickening ability thereof may be decreased due to the hydroxyl group on the surface of the bone graft material. For example, in the case in which carboxymethylcellulose as a thickener is mixed alone with the bone graft material, the carboxymethylcellulose has one functional group. Thus, when the carboxymethylcellulose is mixed with the bone graft material in water or blood, it may be alkalized around the bone graft material and the thickening ability thereof may decrease. In addition, the strength of bonding with the bone graft material may decrease and the carboxymethylcellulose may easily dissolve.

Accordingly, the bone graft composition of the present disclosure contains hydroxypropyl methylcellulose as a crosslinker, in addition to the bone graft material and the thickener, wherein the thickener is carboxymethylcellulose. The hydroxypropyl methylcellulose has two kinds of functional groups: a hydroxyl group and a carboxyl group. Thus, the hydroxypropyl methylcellulose may form covalent bonds with both the bone graft material and the thickener during hydration of the bone graft composition. Thus, when the hydroxypropyl methylcellulose is mixed with the bone graft material and the thickener, it functions as a bonding agent and a barrier due to the excellent bonding strength thereof.

That is, since the thickener contained in the bone graft composition does not easily combine with the bone graft material, it may be combined with the bone graft material using hydroxypropyl methylcellulose as a crosslinker, thereby performing the function thereof. As used herein, "function" means a function of maintaining the volume and internal space of the bone graft composition when a bone defect is filled with the composition, and at the same time, a function of maintaining viscosity and bonding to ensure the convenience of application.

However, in order for the blood or water in the bone defect to flow into the bone graft composition and facilitate new bone formation, the thickener in the bone graft material composition filling the bone defect should be able to dissolve out. Thus, the hydroxypropyl methylcellulose forming a crosslink between the porous bone graft material and the thickener may be limited in the content thereof in the bone graft composition. The limitation of hydroxypropyl methylcellulose according to an embodiment of the present disclosure is applied when the thickener is contained in the bone graft composition.

The thickener that is contained according to the present disclosure is a material having an adhesive property, wherein the thickener is carboxymethylcellulose.

The hydroxypropyl methylcellulose which is contained together with the thickener in the bone graft composition will now be described in detail.

**As** described above, the hydroxypropyl methylcellulose is a material functioning as a crosslinker between the bone graft material and the thickener to form a bone graft composition having excellent procedural performance and bone regeneration ability. According to the Experimental Example to be described below, the change in the volume of the bone graft composition 48 hours after hydration of the bone graft composition was measured, and as a result, it was confirmed that the volume tended to decrease. Furthermore, when the amount of the hydroxypropyl methylcellulose is constant, the decrease rate of the volume tends to decrease as the amount of the thickener increases. This suggests that bonding is enhanced as the amount of the thickener increases. In this Experimental Example, measurement was performed while changing the content (parts by weight) of the hydroxypropyl methylcellulose, and as a result, it was confirmed that there is a critical significance in the content (parts by weight) of the hydroxypropyl methylcellulose as a crosslinker for bonding between the bone graft material and the thickener.

In the Experimental Example on the bone graft composition, the volume reduction rate of the bone graft composition 48 hours after hydration of the composition in an environment that mimics a hydration operation during a medical procedure and a human oral environment was measured, and as a result, the change in the content (parts by weight) of the thickener showed a somewhat insensitive change, but the change in the content (parts by weight) of the hydroxypropyl methylcellulose showed a critical change in a specific content range.

The bone graft composition according to the present disclosure contains the hydroxypropyl methylcellulose in an amount of not less than 0.1 parts by weight and less than 0.3 parts by weight based on 1 part by weight of the bone graft material, together with the bone graft material and the thickener. In this case, it was confirmed that the volume reduction rate of the bone graft composition 48 hours after hydration was substantially about 10% and the bone graft composition had excellent procedural performance and bone regeneration ability.

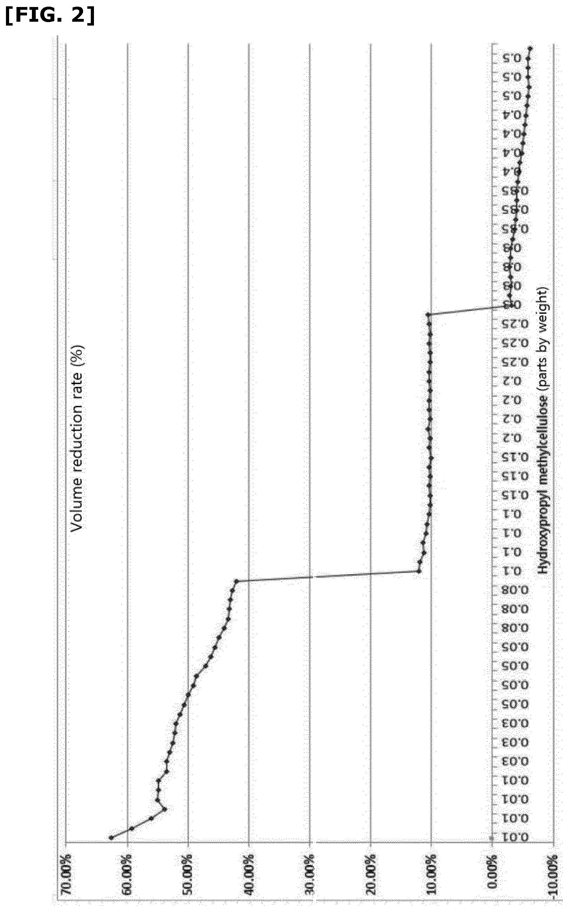

Meanwhile, if the thickener in the bone graft composition is combined with less than 0.1 parts by weight of the hydroxypropyl methylcellulose, the hydroxypropyl methylcellulose does not function as a crosslinker because the amount of the hydroxypropyl methylcellulose is insufficient. This is because the content of the hydroxypropyl methylcellulose is insufficient, so that the thickener having weak bonding strength with the bone graft material cannot be bonded with the bone graft material. Thus, the hydroxypropyl methylcellulose and the thickener cannot properly function as a barrier during hydration, so that the bone graft material and the like are rapidly dissolved and lost. That is, when the bone graft composition is applied to a bone defect, the space of the bone graft material is not maintained and the osteoconductive effect of the composition decreases, and as a result, new bone formation is delayed or difficult. Thus, blood or the like does not properly penetrate the bone graft material, so that blood vessel formation and new bone formation in the bone defect are not properly achieved, indicating that the bone graft composition does not properly function to form bone. This can be confirmed in the Experimental Example to be described later, and can also be confirmed from the fact that the volume reduction rate after hydration is 40% or more at a hydroxypropyl methylcellulose content of less than 0.1 parts by weight as shown in FIG. 2.

When 0.3 parts by weight or more of the hydroxypropyl methylcellulose are combined with the thickener in the bone graft composition, the bonding and barrier role thereof in the bone graft composition rapidly and excessively increases. This excessive enhancement of the bonding and barrier role causes the thickener to not dissolve even by hydration. For this reason, when the bone graft composition is actually implanted into a bone defect, the volume of the bone graft composition does not decrease even after the passage of time, and as a result, the penetration of blood or water into the composition is made difficult. Thus, blood vessel formation and new bone formation in the bone defect are suppressed, indicating that the bone graft composition does not properly function to form bone. This can be confirmed in the Experimental Example to be described later, and can also be confirmed from the fact that the volume reduction rate after hydration decreases rapidly at a hydroxypropyl methylcellulose content of 0.3 parts by weight as shown in FIG. 2.

The bone graft composition according to the present disclosure will now be described in relation to the water absorption property of hydroxypropyl methylcellulose and the covalent bonding between the components in the composition.

If the content of hydroxypropyl methylcellulose is less than 0.1 parts by weight, which is lower than the content (at least 0.1 parts by weight) of the thickener, the covalent bond between the bone graft material and the thickener is not easily formed, and thus the hydroxypropyl methylcellulose cannot properly function as a bonding agent and a barrier. For this reason, when hydration is performed, the thickener having weak bonding strength with the bone graft material dissolves rapidly, resulting in a great reduction in the volume. Therefore, since the space of the bone graft material cannot be maintained at the initial stage of bone grafting, the osteoconductive effect of the bone graft material decreases, and as a result, new bone formation is delayed.

On the other hand, when the hydroxypropyl methylcellulose is added in an amount of 0.1 parts by weight or more based on 1 part by weight of the bone graft material, it may have a water absorption property depending on the proportion of hydroxyapatite, and thus can easily form a covalent bond with the thickener. As can be seen in the Experimental Example to be described later, it can be confirmed that the volume reduction rate significantly changes around 0.1 parts by weight of the hydroxypropyl methylcellulose.

In addition, when the hydroxypropyl methylcellulose in an amount of 0.3 parts by weight or more based on 1 part by weight of the bone graft material is combined with the thickener, a hydrogel is easily formed by a mixture of the bone graft material and the hydroxypropyl methylcellulose, because the water absorption property of the hydroxypropyl methylcellulose is similar to that of hydroxyapatite. When the thickener is additionally used, excessive covalent bonding between the bone graft particles and the thickener occurs while 0.3 parts by weight or more of the hydroxypropyl methylcellulose bonds to the thickener. Thus, since the hydroxypropyl methylcellulose excessively acts as a bonding agent and a barrier, the thickener does not dissolve, and the volume increases rather than decreases. In this case, the volume does not decrease even after the passage of time, and the penetration of blood or water becomes difficult, so that blood vessel formation is not possible and new bone formation is interfered with.

Therefore, the content (parts by weight) of the hydroxypropyl methylcellulose that is contained together with the thickener in the bone graft composition according to the present disclosure is 0.1 to 0.3 parts by weight based on 1 part by weight of the porous bone graft material.

In summary, when the content of the hydroxypropyl methylcellulose in the bone graft composition containing the thickener is not less than 0.1 parts by weight and less than 0.3 parts by weight based on 1 part by weight of the bone graft material, the hydroxypropyl methylcellulose bonds with the thickener, and thus the hydroxypropyl methylcellulose may properly act as a binding agent and a barrier between the bone graft material and the thickener due to viscoelasticity caused by water absorption, thus preventing the dissolution of the thickener and the separation of the bone graft material. Accordingly, since the internal space of the bone graft material may be maintained while the volume thereof may be maintained, and thus the bone graft material has an excellent osteoconductive effect and facilitates new bone formation.

The method for preparing a bone graft composition according to another embodiment of the present disclosure, not falling within the scope of the claimed subject-matter, will now be described.

The method for preparing a bone graft composition according to another embodiment of the present disclosure includes steps of: preparing a bone morphogenetic protein solution by adding a bone morphogenetic protein to a solvent and dissolving the bone morphogenetic protein in the solvent; adsorbing the bone morphogenetic protein onto bone graft material powder; mixing and stirring the bone graft material powder having the bone morphogenetic protein adsorbed thereon, an adhesive thickener, and hydroxypropyl methylcellulose powder as a crosslinking agent capable of bonding with each of the bone graft material powder and the thickener; and freeze-drying the mixture of the bone graft material powder, the thickener and the hydroxypropyl methylcellulose powder, obtained by the mixing and stirring, at low temperature under vacuum. The bone graft composition prepared through these steps may have excellent effects in terms of improving activation of bone formation, biocompatibility, and ease of use.

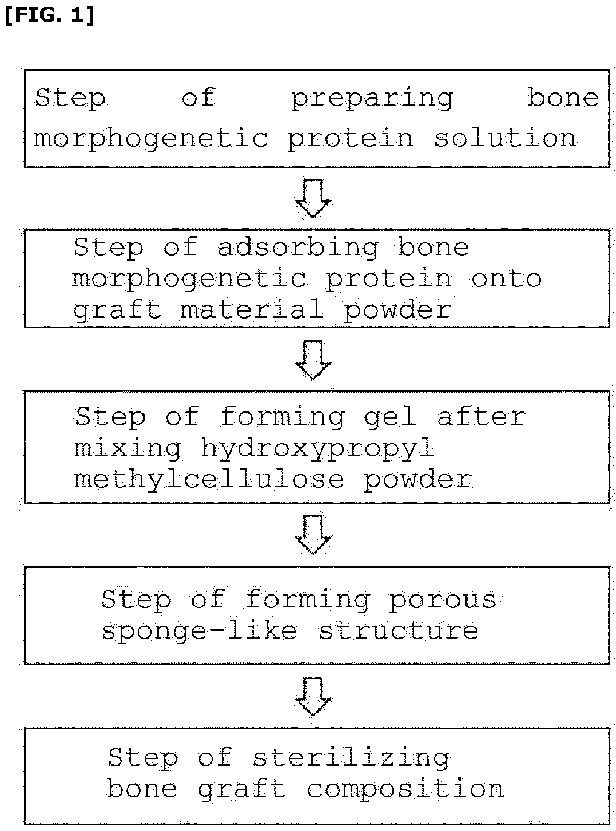

FIG. 1 is a flow diagram schematically showing a method for preparing a bone graft composition according to one embodiment of the present disclosure, not falling within the scope of the claimed subject-matter.

FIG. 2 shows the volume reduction rate depending on the amount (parts by weight) of hydroxypropyl methylcellulose 48 hours after hydration, calculated based on the result data (Table 1) obtained in Experimental Example 1 of the present disclosure.

First, a bone morphogenetic protein solution is prepared by dissolving a bone morphogenetic protein in a solvent. The bone morphogenetic protein solution may be prepared by adding the bone morphogenetic protein to the solvent and dissolving the bone morphogenetic protein in the solvent.

The bone morphogenetic protein may be at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto. Preferably, the bone morphogenetic protein may be rhBMP-2 in terms of the bone formation effect of the present disclosure.

According to one embodiment of the present disclosure, the concentration of the bone morphogenetic protein in the bone morphogenetic protein solution may be 0.05 to 0.15 mg/ml, preferably 0.08 to 0.12 mg/ml. When the concentration of the bone morphogenetic protein is within the above-described range, bone formation by the bone morphogenetic protein may be activated. If the concentration of the bone morphogenetic protein is less than 0.05 mg/ml, the ability of the bone morphogenetic protein to form new bone may be reduced, and if the concentration of the bone morphogenetic protein is more than 0.15 mg/ml, it may cause adverse effects.

In addition, according to one embodiment of the present disclosure, the pH of the bone morphogenetic protein solution may be, for example, 4.6 to 5. When the pH is within the above-described range, bone formation by the bone morphogenetic protein may be activated. If the pH of the bone morphogenetic protein solution is less than 4.6, the ability to form new bone may be reduced, and if the pH of the bone morphogenetic protein solution is more than 5, the ability to form new bone may be reduced. For example, the pH may be adjusted using phosphate buffer saline. When the pH is adjusted using phosphate buffer saline, the effect of forming new bone may be obtained.

Thereafter, the bone morphogenetic protein is adsorbed onto bone graft material powder by soaking the bone graft material powder with the bone morphogenetic protein solution. The previously prepared bone graft material powder may be soaked with the bone morphogenetic protein solution by flushing the bone graft material powder with the bone morphogenetic protein solution or dropping the bone graft material powder into the bone morphogenetic protein solution, whereby the bone morphogenetic protein may be adsorbed onto the bone graft material powder.

The bone graft material powder may be autogenous bone, allogeneic bone, or xenogenic bone. For example, the bone graft material powder may be prepared by placing it in a snap tube.

The average particle diameter (D50) of the bone graft material powder may be 200 to 5,000 µm, preferably 250 to 1,000 µm. If the average particle diameter of the powder is less than 200 µm, the graft material may be absorbed rapidly, and thus osteoconduction required for bone formation may be insufficient, and if the average particle diameter of the powder is more than 5,000 µm, precise processing for application to a patient may be difficult.

According to one embodiment of the present disclosure, the step of adsorbing the bone morphogenetic protein onto the bone graft material powder may include a step of adsorbing the bone morphogenetic protein using a refrigerated centrifuge.

In some cases, the bone morphogenetic protein may also be suspended in the solution. However, when the bone morphogenetic protein is adsorbed while it is rotated using a centrifuge, the bone morphogenetic protein can be prevented from being suspended in the solution, and thus the bone morphogenetic protein may be easily adsorbed onto the surface or into the pores of the bone graft material powder. Only when the bone morphogenetic protein is adsorbed while it is rotated at high speed, it can be prevented from being suspended again after detachment from the bone graft material powder. If the bone morphogenetic protein is rotated at low speed, it can be suspended, and hence cannot be easily adsorbed. Under high-speed rotation, the bone morphogenetic protein can be adsorbed quickly onto the surface or into the pores of the bone graft material powder.

According to one embodiment of the present disclosure, not falling within the scope of the claimed subject-matter, the rotational speed of the refrigerated centrifuge may be 4,000 rpm or more. When the bone morphogenetic protein is adsorbed using the centrifuge, the higher the rotational speed, the better the adsorption. For example, the rotational speed of the centrifuge may be 4,000 rpm or more, and when this rotational speed range is satisfied, the bone morphogenetic protein can be prevented from being suspended in the solution.

According to one embodiment of the present disclosure, not falling within the scope of the claimed subject-matter, the step of adsorbing the bone morphogenetic protein using the refrigerated centrifuge may be performed at a cold temperature of 5°C or below. As the step of adsorbing the bone morphogenetic protein using the refrigerated centrifuge is performed at a cold temperature of 5°C or below, it is possible to maximize the effect of adsorbing the bone morphogenetic protein onto the surface or into the pores of the bone graft material powder through rotation while preventing the denaturation of the bone morphogenetic protein that is weak to heat, by preventing the temperature of the solution from being increased due to rotation. The cold temperature may be a temperature at which the solution does not freeze. For example, the cold temperature may be 5°C or below, preferably 0.5 to 1.5°C.

Thereafter, the bone graft material powder having the bone morphogenetic protein adsorbed thereon, the thickener and hydroxypropyl methylcellulose powder are mixed together and stirred to form a gel. The viscous gel thus formed can improve the adhesion of the bone graft material powder. For example, the stirring may be performed using a mixer. As the graft material powder is stirred with the hydroxypropyl methylcellulose in powder form together with the thickener, a product with homogeneous quality can be obtained.

Thereafter, the mixture of the bone graft material powder, the thickener and the hydroxypropyl methylcellulose powder, obtained by the mixing and stirring process, is freeze-dried at low temperature under vacuum to form a mixture having a porous structure. A sponge-like structure containing a plurality of pores may also be formed by freeze-drying the mixture of the bone graft material powder, the thickener and the hydroxypropyl methylcellulose powder, obtained by the mixing and stirring process, at a low temperature under vacuum.

A sponge-like structure including a porous structure may be formed by the freeze-drying treatment under vacuum. The gel may be absorbed into the bone graft material powder to form a sponge-like structure including a porous structure, and it is believed that the treatment under vacuum mainly contributes to the formation of the sponge-like structure including a porous structure.

The method for preparing a bone graft composition according to one embodiment of the present disclosure, not falling within the scope of the claimed subject-matter, may further include a packaging step.

The method for preparing a bone graft composition according to one embodiment of the present disclosure, not falling within the scope of the claimed subject-matter, may further include a step of placing the prepared bone graft composition including a sponge-like structure containing a plurality of pores in a snap tube sized to be inserted into a syringe. When the method further includes the step of placing the composition in a snap tube sized to be inserted into a syringe, the composition may be sized to be inserted into the syringe and thus may be inserted directly into the syringe without a separate process, so that the operation of the process for preparing the bone graft composition can be facilitated.

The method for preparing a bone graft composition according to an embodiment of the present disclosure disclosure, not falling within the scope of the claimed subject-matter, may further include a step of placing and sealing the bone graft composition including a sponge-like structure containing a plurality of pores, placed in the snap tube, in a syringe. When the bone graft composition is provided in the syringe, it is possible to ensure ease of use and significantly reduce the possibility of contamination that may occur during use.

The method for preparing a bone graft composition according to one embodiment of the present disclosure, not falling within the scope of the claimed subject-matter, may further include a step of sterilizing the composition.

In one embodiment of the present disclosure, the bone graft composition including a sponge-like structure containing a plurality of pores may be sterilized by ethylene oxide gas. For example, the concentration of the ethylene oxide gas may be 450 to 1,200 mg/l.

If the concentration of the ethylene oxide gas is less than 450 mg/l, sterilization may be insufficient, and if the concentration of the ethylene oxide gas is more than 1,200 mg/l, denaturation of the bone morphogenetic protein may occur.

According to one embodiment of the present disclosure, the bone graft composition including a sponge-like structure containing a plurality of pores may be sterilized by gamma-ray irradiation. For example, the dose of the gamma-ray irradiation may be 10 to 25 kGy. If the dose of the gamma-ray irradiation is less than 10 kGy, sterilization may be insufficient, and if the dose of the gamma-ray irradiation is more than 25 kGy, denaturation of the bone morphogenetic protein may occur.

In order to apply the bone graft composition prepared according to the above-described method to the human body (e.g., teeth), the bone graft composition should have viscosity and, at the same time, the internal space (shape) thereof should be maintained, so that blood penetration and new bone formation in the composition is possible. This viscosity and space-maintaining property may be determined by the content of hydroxypropyl methylcellulose or the like in the bone graft composition. Furthermore, when hydroxypropyl methylcellulose and the thickener are contained together in the bone graft composition, the viscosity and the space-maintaining property may be achieved by a lower content of the hydroxypropyl methylcellulose.

For example, in the case in which the bone graft composition is applied to teeth, when a dental operator applies the bone graft composition to a missing tooth, the hydroxypropyl methylcellulose contained in the bone graft composition should reach a certain dissolution rate or more within a certain time so that the composition can be applied to the missing tooth with an appropriate adhesion and can fit the shape of the missing tooth during a medical procedure. After the bone graft composition is applied to the missing tooth during the medical procedure, the phenomenon that the bone graft composition flows out around or detaches should not occur, and a phenomenon should also not occur in which the hydroxypropyl methylcellulose in the bond graft composition does not dissolve so that the bone graft composition cannot adhere to the bone defect portion, making it impossible to perform the medical procedure. Therefore, the bone graft composition requires critical conditions of hydroxypropyl methylcellulose, and the function of the bone graft material may differ depending on the content of the hydroxypropyl methylcellulose.

As can be seen from the Experimental Example of the present disclosure to be described later, when the content of the hydroxypropyl methylcellulose in the bone graft composition is not less than 0.1 parts by weight and less than 0.3 parts by weight, the hydroxypropyl methylcellulose bonds with the thickener, and thus the hydroxypropyl methylcellulose may properly act as a binding agent and a barrier between the bone graft material and the thickener due to viscoelasticity caused by water absorption, thus preventing the dissolution of the thickener and the separation of the bone graft material. Accordingly, since the internal space of the bone graft material may be maintained while the volume thereof may be maintained, and thus the bone graft material has an excellent osteoconductive effect and facilitates new bone formation.

The thickener that is contained according to the present disclosure is a material having an adhesive property, and is carboxymethylcellulose.

Hereinafter, preferred examples will be presented to help the understanding of the present disclosure. However, these examples are merely to illustrate the present disclosure and are not intended to limit the scope of the present disclosure as defined in the appended claims.

### Experimental Examples

### - Experiment for Examining Volume Reduction Rate Depending on Content (Parts by Weight) of Hydroxypropyl Methylcellulose

A thickener and hydroxypropyl methylcellulose were added to 0.25 g of a bone graft material while changing the amount of the hydroxypropyl cellulose within a range of 0.01 to 0.5 parts by weight as shown in Table 1 below. For each amount (parts by weight) of the hydroxypropyl methylcellulose, the amount of the thickener was changed from 0.1 parts by weight to 1 part by weight. As the thickener, carboxymethylcellulose was used. Other materials having a thickening effect may also be used as the thickener. Each of the mixtures of the bone graft material, the thickener and the hydroxypropyl methylcellulose was dissolved in a solvent (water), and then immediately, the volume of each mixture was measured. In addition, the volume of each mixture after 48 hours was measured. The amount of the solvent (water) corresponds to an optimal degree of hydration to which the mixture can dissolve well. In this Experiment, the amount of the solvent was 1 to 1.5 times, for example, 1.2 times, the total weight of the mixture.

**[Table 1]**

| hydroxypropyl methylcellulose (parts by weight) | Thickener (parts by weight) | Initial volume (cc) | Volume (cc) after 48 hours | Volume reduction rate (%) |
|---|---|---|---|---|
| 0.01 | 0.1 | 0.270 | 0.101 | 62.63% |
| 0.01 | 0.2 | 0.300 | 0.122 | 59.33% |
| 0.01 | 0.3 | 0.330 | 0.145 | 56.06% |
| 0.01 | 0.5 | 0.390 | 0.180 | 53.85% |
| 0.01 | 0.7 | 0.450 | 0.202 | 55.11% |
| 0.01 | 0.9 | 0.510 | 0.230 | 54.90% |
| 0.01 | 1 | 0.520 | 0.235 | 54.81% |
| 0.03 | 0.1 | 0.280 | 0.130 | 53.57% |
| 0.03 | 0.2 | 0.310 | 0.144 | 53.55% |
| 0.03 | 0.3 | 0.340 | 0.160 | 52.94% |
| 0.03 | 0.5 | 0.400 | 0.190 | 52.50% |
| 0.03 | 0.7 | 0.460 | 0.220 | 52.17% |
| 0.03 | 0.9 | 0.520 | 0.250 | 51.92% |
| 0.03 | 1 | 0.530 | 0.258 | 51.32% |
| 0.05 | 0.1 | 0.300 | 0.148 | 50.67% |
| 0.05 | 0.2 | 0.320 | 0.160 | 50.00% |
| 0.05 | 0.3 | 0.350 | 0.178 | 49.14% |
| 0.05 | 0.5 | 0.410 | 0.211 | 48.54% |
| 0.05 | 0.7 | 0.470 | 0.249 | 47.02% |
| 0.05 | 0.9 | 0.530 | 0.285 | 46.23% |
| 0.05 | 1 | 0.550 | 0.299 | 45.64% |
| 0.08 | 0.1 | 0.330 | 0.182 | 44.85% |
| 0.08 | 0.2 | 0.350 | 0.196 | 44.00% |
| 0.08 | 0.3 | 0.380 | 0.215 | 43.42% |
| 0.08 | 0.5 | 0.440 | 0.250 | 43.18% |
| 0.08 | 0.7 | 0.500 | 0.285 | 43.00% |
| 0.08 | 0.9 | 0.560 | 0.321 | 42.68% |
| 0.08 | 1 | 0.580 | 0.336 | 42.07% |
| 0.1 | 0.1 | 0.350 | 0.308 | 12.00% |
| 0.1 | 0.2 | 0.370 | 0.326 | 11.89% |
| 0.1 | 0.3 | 0.390 | 0.346 | 11.28% |
| 0.1 | 0.5 | 0.410 | 0.363 | 11.46% |
| 0.1 | 0.7 | 0.430 | 0.383 | 10.93% |
| 0.1 | 0.9 | 0.450 | 0.402 | 10.67% |
| 0.1 | 1 | 0.470 | 0.421 | 10.43% |
| 0.15 | 0.1 | 0.400 | 0.359 | 10.25% |
| 0.15 | 0.2 | 0.420 | 0.377 | 10.24% |
| 0.15 | 0.3 | 0.440 | 0.394 | 10.45% |
| 0.15 | 0.5 | 0.460 | 0.413 | 10.22% |
| 0.15 | 0.7 | 0.480 | 0.430 | 10.42% |
| 0.15 | 0.9 | 0.500 | 0.450 | 10.00% |
| 0.15 | 1 | 0.520 | 0.466 | 10.38% |
| 0.2 | 0.1 | 0.450 | 0.404 | 10.22% |
| 0.2 | 0.2 | 0.470 | 0.420 | 10.64% |
| 0.2 | 0.3 | 0.490 | 0.440 | 10.20% |
| 0.2 | 0.5 | 0.510 | 0.457 | 10.39% |
| 0.2 | 0.7 | 0.530 | 0.475 | 10.38% |
| 0.2 | 0.9 | 0.550 | 0.494 | 10.18% |
| 0.2 | 1 | 0.570 | 0.511 | 10.35% |
| 0.25 | 0.1 | 0.500 | 0.448 | 10.40% |
| 0.25 | 0.2 | 0.520 | 0.467 | 10.19% |
| 0.25 | 0.3 | 0.540 | 0.485 | 10.19% |
| 0.25 | 0.5 | 0.560 | 0.502 | 10.36% |
| 0.25 | 0.7 | 0.580 | 0.521 | 10.17% |
| 0.25 | 0.9 | 0.600 | 0.538 | 10.33% |
| 0.25 | 1 | 0.620 | 0.555 | 10.48% |
| 0.3 | 0.1 | 0.550 | 0.567 | -3.09% |
| 0.3 | 0.2 | 0.570 | 0.586 | -2.81% |
| 0.3 | 0.3 | 0.590 | 0.608 | -3.05% |
| 0.3 | 0.5 | 0.610 | 0.628 | -2.95% |
| 0.3 | 0.7 | 0.630 | 0.648 | -2.86% |
| 0.3 | 0.9 | 0.650 | 0.669 | -2.92% |
| 0.3 | 1 | 0.670 | 0.690 | -2.99% |
| 0.35 | 0.1 | 0.600 | 0.620 | -3.33% |
| 0.35 | 0.2 | 0.620 | 0.643 | -3.71% |
| 0.35 | 0.3 | 0.640 | 0.664 | -3.75% |
| 0.35 | 0.5 | 0.660 | 0.686 | -3.94% |
| 0.35 | 0.7 | 0.680 | 0.707 | -3.97% |
| 0.35 | 0.9 | 0.700 | 0.728 | -4.00% |
| 0.35 | 1 | 0.720 | 0.750 | -4.17% |
| 0.4 | 0.1 | 0.650 | 0.678 | -4.31% |
| 0.4 | 0.2 | 0.670 | 0.700 | -4.48% |
| 0.4 | 0.3 | 0.690 | 0.723 | -4.78% |
| 0.4 | 0.5 | 0.710 | 0.745 | -4.93% |
| 0.4 | 0.7 | 0.730 | 0.768 | -5.21% |
| 0.4 | 0.9 | 0.750 | 0.790 | -5.33% |
| 0.4 | 1 | 0.770 | 0.812 | -5.45% |
| 0.5 | 0.1 | 0.700 | 0.740 | -5.71% |
| 0.5 | 0.2 | 0.720 | 0.762 | -5.83% |
| 0.5 | 0.3 | 0.740 | 0.784 | -5.95% |
| 0.5 | 0.5 | 0.760 | 0.804 | -5.79% |
| 0.5 | 0.7 | 0.780 | 0.825 | -5.77% |
| 0.5 | 0.9 | 0.800 | 0.847 | -5.87% |
| 0.5 | 1 | 0.820 | 0.870 | -6.10% |

As shown in Table 1 above, it can beconfirmed that the volume reduction rate after 48 hours decreased as the amount of the hydroxypropyl methylcellulose increased. In addition, it can be confirmed that, at the same amount (parts by weight) of the hydroxypropyl methylcellulose, the volume reduction rate decreased as the amount (parts by weight) of the thickener increased. Here, the amount (parts by weight) of the hydroxypropyl methylcellulose has threshold values of 0.1 and 0.3. A volume reduction rate corresponding to a negative value indicates that the bone graft composition absorbed water, so that the volume thereof increased than decreased.

FIG. 2 shows the volume reduction rate of the composition as a function of the amount (parts by weight) of hydroxypropyl methylcellulose (x-axis), calculated based on the result data (Table 1) obtained in the Experimental Example. A greater value on the y-axis indicates a greater reduction in the volume, and a smaller value on the y-axis indicates a smaller reduction in the volume.

When the thickener and the hydroxypropyl methylcellulose are contained together, the volume reduction rate of the bone graft composition within 48 hours may be about 10%, which is shown when the content (parts by weight) of the hydroxypropyl methylcellulose in the bone graft composition is not less than 0.1 parts by weight and less than 0.3 parts by weight. Therefore, the hydroxypropyl methylcellulose forms a bond with the thickener, and thus the hydroxypropyl methylcellulose may properly act as a binding agent and a barrier between the bone graft material and the thickener due to viscoelasticity caused by water absorption, thus preventing the dissolution of the thickener and the separation of the bone graft material. Accordingly, since the internal space of the bone graft material may be maintained while the volume thereof may be maintained, and thus the bone graft material has an excellent osteoconductive effect and may facilitate new bone formation.

As described above, the bone graft composition according to the present disclosure has excellent procedural performance by containing the thickener, which has an adhesive property, and hydroxypropyl methylcellulose as a crosslinker capable of bonding with each of the bone graft material and the thickener.

In addition, the bone graft composition according to the present disclosure has viscosity and an internal space-maintaining property, and at the same time, blood or the like easily flows into the bone graft composition, so that the composition has excellent bone regeneration ability, because the bone graft composition contains the thickener, which has an adhesive property, and hydroxypropyl methylcellulose as a crosslinker capable of bonding with each of the bone graft material and the thickener.

## Claims

1. A bone graft composition containing:
a bone graft material;
a thickener having an adhesive property, wherein the thickener is carboxymethylcellulose;
wherein the content of the hydroxypropyl methylcellulose is not less than 0.1 parts by weight or less and less than 0.3 parts by weight based on 1 part by weight of the bone graft material, and
the content of the thickener is 0.1 parts by weight to 1 part by weight based on 1 part by weight of the bone graft material; and
hydroxypropyl methylcellulose as a crosslinking agent capable of bonding with each of the bone graft material and the thickener.

2. The bone graft composition of claim 1, wherein, when the bone graft composition is hydrated for application to a bone defect, the volume thereof is reduced.

3. The bone graft composition of claim 1, wherein, when the bone graft composition is hydrated for application to a bone defect, the volume thereof is reduced by more than 0% and less than 40% at 48 hours after the hydration.

4. The bone graft composition of claim 1, wherein, when the bone graft composition is hydrated for application to a bone defect, the volume thereof is reduced by 10% at 48 hours after the hydration.

## Patentansprüche

1. Eine Knochenersatzzusammensetzung, die Folgendes enthält:
ein Knochenersatzmaterial;
ein Verdickungsmittel mit Klebeeigenschaften, wobei das Verdickungsmittel Carboxymethylcellulose ist;
wobei der Gehalt an Hydroxypropylmethylcellulose nicht weniger als 0,1 Gewichtsteile oder weniger und weniger als 0,3 Gewichtsteile, bezogen auf 1 Gewichtsteil des Knochenersatzmaterials, beträgt und der Gehalt an Verdickungsmittel 0,1 Gewichtsteile bis 1 Gewichtsteil, bezogen auf 1 Gewichtsteil des Knochenersatzmaterials, beträgt; und
Hydroxypropylmethylcellulose als Vernetzungsmittel, das in der Lage ist, sich mit dem Knochenersatzmaterial und dem Verdickungsmittel zu verbinden.

2. Knochenersatzzusammensetzung nach Anspruch 1, wobei sich das Volumen der Knochenersatzzusammensetzung verringert, wenn sie zur Anwendung auf einen Knochendefekt hydratisiert wird.

3. Die Knochenersatzzusammensetzung nach Anspruch 1, wobei, wenn die Knochenersatzzusammensetzung zur Anwendung auf einen Knochendefekt hydratisiert wird, ihr Volumen 48 Stunden nach der Hydratisierung um mehr als 0 % und weniger als 40 % reduziert ist.

4. Die Knochenersatzzusammensetzung nach Anspruch 1, wobei das Volumen der Knochenersatzzusammensetzung, wenn sie zur Anwendung auf einen Knochendefekt hydratisiert wird, 48 Stunden nach der Hydratisierung um 10 % reduziert ist.

## Revendications

1. Composition de greffe osseuse, contenant :
un matériau de greffe osseuse ;
un épaississant ayant une propriété adhésive, ledit épaississant étant la carboxyméthylcellulose ;
la teneur en hydroxypropylméthylcellulose étant supérieure ou égale à 0,1 partie en poids et inférieure à 0,3 partie en poids sur la base de 1 partie en poids du matériau de greffe osseuse, et
la teneur en épaississant étant comprise entre 0,1 partie en poids et 1 partie en poids sur la base de 1 partie en poids du matériau de greffe osseuse ; et
de l'hydroxypropylméthylcellulose en tant qu'agent de réticulation apte à se lier au matériau de greffe osseuse ainsi qu'à l'épaississant.

2. Composition de greffe osseuse selon la revendication 1, où, lorsque la composition de greffe osseuse est hydratée pour une application sur une lésion osseuse, le volume de celle-ci est réduit.

3. Composition de greffe osseuse selon la revendication 1, où, lorsque la composition de greffe osseuse est hydratée pour une application sur une lésion osseuse, le volume de celle-ci est réduit de plus de 0 % et de moins de 40 % 48 heures après l'hydratation.

4. Composition de greffe osseuse selon la revendication 1, où, lorsque la composition de greffe osseuse est hydratée pour une application sur une lésion osseuse, le volume de celle-ci est réduit de 10 % 48 heures après l'hydratation.
